# EUROPEAN PATENT APPLICATION

(11) **EP 4 740 746 A1**
(43) Date of publication of application: **13.05.2026**
(21) Application number: 24835859.0
(22) Date of filing: 12.06.2024
(51) Int. Cl.: A23L 5/00, A23J 3/26, A23L 33/185, A61K 9/48, A61K 47/10, A61K 47/14, A61K 47/18, A61K 47/42

(54) **COMPOSITION FOR PRODUCING CAPSULE FILM, METHOD FOR PRODUCING MATERIAL FOR PRODUCING CAPSULE FILM, AND CAPSULE FILM**

(30) Priority: 03.07.2023 JP 2023109071
(71) Applicant: Sunsho Pharmaceutical Co., Ltd., Fuji-shi, Shizuoka 419-0201 (JP)
(72) Inventor: TSUNODA, Yamato, Fuji-shi, Shizuoka 419-0201 (JP); YANAGIHARA, Aoi, Fuji-shi, Shizuoka 419-0201 (JP); HIRASAWA, Wataru, Fuji-shi, Shizuoka 419-0201 (JP)
(74) Representative: TBK
(86) International application number: PCT/JP2024/021282
(87) International publication number: WO 2025/009342

(57) **Abstract**

An object of the present invention is to enhance the formability of a capsule film material containing plant-based protein (for example, a soft capsule film material) to impart novel functions to capsule formulations. Examples of the novel functions include that the capsule film itself exhibits various biological effects, that a system that delivers the active substance of a capsule formulation to intestines, in particular, the large intestine (DDS: Drug Delivery System) is realized, and that a highly active drug is used as the content, etc. As a solving means, there is provided a composition for producing a capsule film, the composition containing at least one type selected from the group consisting of polyols, triesters, and trialkylamino acids, and a plant-derived protein. The composition for producing a capsule film is subjected to pressurized heat treatment to prepare a material for producing a capsule film, and the material for producing a capsule film can be formed into a capsule of a capsule formulation (preferably, a soft capsule formulation).

## Description

### Technical Field

The present invention relates to a composition for producing a capsule film, a method for producing a material for producing a capsule film, and a capsule film.

### Background Art

The underlying pathological conditions of age-associated diseases, such as malignant neoplasms and COPD, and non-communicable diseases (NCD) have been reported to be associated with chronic inflammation, such as increased levels of blood inflammatory markers such as inflammatory cytokines, etc. From the viewpoints of extending healthy life expectancy and preventing care, the importance of preventing and alleviating chronic inflammation is increasing, and nutritional intake focusing on the anti-inflammatory effects while being based on the intake of sufficient energy, high-quality proteins, vitamins, and minerals has become necessary.

Under such circumstances, NPL 1 reported that an anti-inflammatory effect by the TNF-α lowering effect was confirmed among elderly people aged 70 or over living in low-cost nursing homes as a result of taking soybean protein, which is a plant-based protein, for a certain period of time. In addition, plant-based proteins have been confirmed to have various physiological activities.

Furthermore, it is known that hydrolysis of a protein makes the protein indigestible. Hydrolysis of a protein is done by heat treatment (including pressurized heat treatment), enzyme treatment, acid treatment, or the like. Techniques for making proteins indigestible have also been reported, and, for example, PTL 1 describes that a protein-containing plant-derived material is subjected to proteolytic enzyme treatment to produce an indigestible-component-rich raw material.

Indigestible proteins are also referred to as resistant proteins or the like, and are sometimes categorized as one of Luminacoids (fermentable dietary fibers). Indigestible proteins are rarely hydrolyzed by digestive enzymes in the stomach and the small intestine and can be delivered to the large intestine. Thus, indigestible proteins are known to exhibit dietary fiber-like physiological functions, and are reported to have a cholesterol lowering effect, a bowel movement improvement effect, etc. (PTL 2).

### Citation List

### Patent Literature

PTL 1: Japanese Unexamined Patent Application Publication No. 2008-189625
PTL 2: Japanese Unexamined Patent Application Publication No. 2019-129737

### Non Patent Literature

NPL 1: Japan Plant Protein Foods Association Research Report "Nutritional effects of intake of plant-based protein on extending healthy life expectancy of elderly people" [online], November 2020 [retrieved: June 2, 2023], Internet <URL: https://www.protein.or.jp/wp-content/themes/protein.or.jp/pdf/study3.pdf>

### Summary of Invention

### Technical Problem

Under such situations, plant-based proteins are expected to be adopted to various applications as food, beverages, and pharmaceuticals that have health promoting effects.

However, compositions that contain plant-based proteins have low formability in general, and preparing the formulation thereof has been difficult in some cases. Thus, an object of the present invention is to enhance the formability of a capsule film material containing a plant-based protein (for example, a soft capsule film material) to impart novel functions to capsule formulations. For example, by allowing a capsule film of a capsule formulation to contain a plant-based protein, the capsule film itself is expected to exhibit various biological effects. In addition, it is anticipated that, by making the plant-based protein contained in the capsule film material indigestible, a system (DDS: Drug Delivery System) that delivers the active substance of the capsule formulation to intestines, in particular, the large intestine, would be realized. Furthermore, it is anticipated that, by making the plant-based protein contained in the capsule film material indigestible, a capsule formulation that encapsulates highly reactive content (highly active substance) in a capsule film would be provided.

### Solution to Problem

The present invention relates to, for example, a composition for producing a capsule film.
[1] A composition for producing a capsule film, the composition containing at least one type (these may also generically be referred to as the "formability imparting component") selected from the group consisting of polyols, triesters, and trialkylamino acids, and a plant-derived protein.
[2] The composition described in [1] above, in which the content mass ratio of the formability imparting component to the plant-derived protein is 25:75 to 80:20.
[3] The composition described in [1] or [2] above, in which a content ratio of water is less than 20 mass%.
[4] The composition described in any one of [1] to [3] above, in which the plant-derived protein includes at least one type selected from the group consisting of soybean-derived proteins, adzuki bean-derived proteins, kidney bean-derived proteins, cacao bean-derived proteins, wheat-derived proteins, buckwheat-derived proteins, and rice-derived proteins.
[5] The composition described in any one of [1] to [4] above, in which the formability imparting component includes glycerin.

In addition, the present invention relates to, for example, a method for producing a composition for producing a capsule film.
[6] A method for producing a material for producing a capsule film, the method including a step of pressurizing and heating a composition containing at least one type (these may also generically be referred to as the "formability imparting component") selected from the group consisting of polyols, triesters, and trialkylamino acids, and a plant-derived protein.
[7] The method for producing described in [6] above, in which a content mass ratio of the formability imparting component to the plant-derived protein is 25:75 to 80:20.
[8] The method for producing described in [6] or [7] above, in which a content ratio of water is less than 20 mass%.
[9] The method for producing described in any one of [6] to [8] above, in which the plant-derived protein includes at least one type selected from the group consisting of soybean-derived proteins, adzuki bean-derived proteins, kidney bean-derived proteins, cacao bean-derived proteins, wheat-derived proteins, buckwheat-derived proteins, and rice-derived proteins.
[10] The method for producing described in any one of [6] to [9] above, in which the formability imparting component includes glycerin.
[11] The method for producing described in any one of [6] to [10] above, in which the heating temperature is 50 to 200°C.

Furthermore, the present invention relates to, for example, a capsule film and a capsule formulation that includes the capsule film.
[12] A capsule film including at least one type (these may also generically be referred to as the "formability imparting component") selected from the group consisting of polyols, triesters, and trialkylamino acids, and a plant-derived protein.
[13] The capsule film described in [12] above, in which a content mass ratio of the formability imparting component to the plant-derived protein is 25:75 to 80:20.
[14] The capsule film described in [12] or [13] above, in which the plant-derived protein includes at least one type selected from the group consisting of soybean-derived proteins, adzuki bean-derived proteins, kidney bean-derived proteins, cacao bean-derived proteins, wheat-derived proteins, buckwheat-derived proteins, and rice-derived proteins.
[15] The capsule film described in any one of [12] to [14] above, in which the formability imparting component includes glycerin.
[16] The capsule film described in any one of [12] to [15] above, in which the plant-derived protein has been subjected to pressurized heat treatment.
[17] A capsule formulation including the capsule film described in any one of [12] to [16] above, and a content encapsulated in the capsule film.
[18] A method for reducing digestibility of a protein, the method including a step of pressurizing and heating a composition containing the protein.
[19] The method described in [18] above, wherein the composition further contains a polyol.

### Advantageous Effects of Invention

According to the present invention, novel applications of plant-derived protein capsule films are provided. Furthermore, according to the present invention, indigestible capsule films are obtained. In addition, according to the present invention, capsule formulations having novel functions are provided.

### Description of Embodiments

Hereinafter, embodiments for implementing the present invention are described in detail.

### [1. Composition for producing a capsule film]

A composition for producing a capsule film of one embodiment of the present invention is a composition that is prepared as a material for producing a capsule film by being processed or treated (typically involving pressurized heat treatment but not limited to this). A composition for producing a capsule film at least contains at least one type (these may also be generically referred to as the "formability imparting component") selected from the group consisting of triols, triesters, and trialkylamino acids, and a plant-derived protein; and may contain any other desired components. In the description below, each of the components of the composition for producing a capsule film is described.

### [1-1. Plant-derived protein]

The plant-derived protein contained in the composition for producing a capsule film of the present invention is not particularly limited as long as the protein is derived from a plant. Examples of the plant from which the protein is derived include grains, beans, nuts, tuber crops, vegetables, fruits, mushrooms, algae, etc. Examples of the grains are wheat, barley, oats, rice, corn, buckwheat, etc.; examples of the beans are soybeans, peas, adzuki beans, chickpeas, lentils, fava beans, mung beans, lupin beans, kidney beans, cacao beans, etc.; examples of the nuts are almonds, peanuts, cashews, pistachios, hazelnuts, macadamia nuts, flaxseed, sesame, rapeseed, cottonseed, safflower, common sunflower, etc.; examples of the tuber crops are potatoes, sweet potatoes, mountain yams, Jerusalem artichokes, cassava, etc.; examples of the vegetables are asparagus, artichokes, cauliflower, broccoli, edamame beans, etc.; examples of the fruits are bananas, jackfruit, kiwifruit, coconut, avocado, olives, etc.; examples of the mushrooms are button mushrooms, king oyster mushrooms, shiitake mushrooms, shimeji mushrooms, maitake mushrooms, etc.; and examples of the algae are chlorella, spirulina, euglena, seaweed, kelp, wakame, hijiki, tengusa, mozuku, etc.

The plant-derived protein in the present invention includes proteins contained in fermented products obtained from plants as raw materials (for example, as rice-derived proteins, proteins contained in sake cake obtained from rice as a raw material) and denatured products of proteins collected from plants.

When the composition for producing a capsule film of the present invention is prepared as a material for producing a capsule film, the plant-derived protein contained in the composition for producing a capsule film is preferably made indigestible by performing pressurized heat treatment or the like (as a result, indigestibility can be imparted to the capsule film). Examples of the plant-derived proteins that can be easily made indigestible include soybean-derived proteins, adzuki bean-derived proteins, kidney bean-derived proteins, cacao bean-derived proteins, wheat-derived proteins, buckwheat-derived proteins, rice-derived proteins, etc. The plant-derived proteins can be used alone or can be used in combination of two or more types.

The lower limit of the content amount of the plant-derived protein in the composition for producing a capsule film of the present invention relative to the total amount of the composition is preferably 10 mass%, more preferably 15 mass%, and particularly preferably 20 mass%. By containing the plant-derived protein, the obtained capsule film can be imparted with specified functions (including physiologically active functions and indigestibility by a plant-derived protein, etc.). The upper limit of the content amount of the plant-derived protein in the composition for producing a capsule film of the present invention is not particularly limited, but, in view the formability thereof, the upper limit relative to the total amount of the composition is preferably 85 mass%, more preferably 80 mass%, and particularly preferably 75 mass%.

### [1-2. Formability imparting component]

The composition for producing a capsule film of the present invention becomes a material for producing a capsule film by being processed; and the formability imparting component contained in the composition for producing a capsule film can impart good formability to the material for producing a capsule film. The formability imparting component can be at least one type selected from polyols, triesters, and trialkylamino acids.

### [1-2-1. Polyols]

Among of the formability imparting components, polyols may be any organic compound containing multiple hydroxy groups, and can be a diol, a triol, or a polyol having 4 or more hydroxy groups. The polyols can be used alone or can be used in combination of two or more types.

The diol can be, for example, (poly)alkylene glycol, a fatty acid monoester of glycerin, or the like. Examples of (poly)alkylene glycol are ethylene glycol, propylene glycol, polyethylene glycol, polypropylene glycol, etc. Examples of the fatty acid monoester of glycerin are glyceryl monocaprylate, glyceryl monobehenate, glyceryl monopalmitate, glyceryl monostearate, glyceryl monobehenate, etc.

The triol can be, for example, glycerin, a fatty acid monoester of diglycerin, or the like. Examples of the fatty acid monoester of diglycerin include a stearic acid monoester of diglycerin, etc.

Examples of the polyol having 4 or more hydroxy groups are chain sugar alcohols such as erythritol, xylitol, sorbitol, mannitol, etc.; chain compounds having 4 or more hydroxy groups, such as polyglycerin; sugar alcohols including ring structures such as isomalt, lactitol, maltitol, etc.; and polysaccharides such as trehalose, cellulose derivatives, chitosan, etc. Examples of the cellulose derivatives include methyl cellulose, hydroxypropyl cellulose, hydroxypropylmethyl cellulose, carboxymethyl cellulose, ethyl cellulose having a low ethoxylation rate (preferably, an ethoxylation rate of 40 mass% or less), etc.

### [1-2-2. Triesters]

The formability imparting component may be a triester. Examples of the triester are a carboxylic acid triesters of glycerin such as triacetin, glycerol tripropionate, glycerol tributyrate, glycerol trihexanoate, etc.; citric acid triesters such as triethyl citrate, triethyl acetylcitrate, tributyl citrate, triethyl acetylcitrate, tris(2-ethylhexyl) acetylcitrate, etc.; and trimellitic acid triesters such as tributyl trimellitate, tris(2-ethylhexyl) trimellitate, etc. The triesters can be used alone or can be used in combination of two or more types.

### [1-2-3. Trialkylamino acids]

The formability imparting component may be a trialkylamino acid. The trialkylamino acid is not particularly limited as long as it is a compound that contains an N,N,N-trialkylammonio group and -COO- group or a carboxy group, or a salt thereof. Examples of the trialkylamino acid include betaine(trimethylglycine), triethylglycine, tripropylglycine, triisopropylglycine, trimethyl-γ-aminobutyric acid, N,N,N-trimethylalanine, N,N,N-triethylalanine, N,N,N-triisopropylalanine, N,N,N-trimethylmethylalanine, carnitine and acetylcarnitine, and salts thereof (for example, hydrochlorides and organic acid salts), etc. The trialkylamino acids can be used alone or can be used in combination of two or more types.

The composition for producing a capsule film of the present invention preferably contains a polyol as the formability imparting component. Furthermore, from the point of enhancing the formability of the material for producing a capsule film, the composition for producing a capsule film preferably contains glycerin as the formability imparting component. The content ratio of glycerin in the composition for producing a capsule film relative to the total of the formability imparting component is preferably 30 mass% or more and particularly preferably 50 mass% or more.

The lower limit of the content amount of the formability imparting component in the composition for producing a capsule film of the present invention relative to the total amount of the composition is preferably 10 mass%, more preferably 15 mass%, and yet more preferably 25 mass%. This is to impart sufficient formability to the material for producing a capsule film. Meanwhile, the upper limit of the content amount of the formability imparting component in the composition for producing a capsule film relative to the total amount of the composition is preferably 90 mass%, more preferably 85 mass%, and yet more preferably 80 mass%.

In addition, in the composition for producing a capsule film of the present invention, the content mass ratio of the formability imparting component to the plant-derived protein (total mass of formability imparting component: total mass of plant-derived protein) is preferably 25:75 to 80:20, more preferably 30:70 to 80:20, yet more preferably 35:65 to 80:20, and particularly preferably 40:60 to 75:25. By adjusting the content ratio of the two components, a material having good formability for producing a capsule film can be obtained from the composition for producing a capsule film.

### [1-3. Other components]

The composition for producing a capsule film of the present invention can contain, if necessary, other base ingredients, solvents, thickeners, pH adjusters, metal sequestering agents, film coloring agents, flavoring agents, sweeteners, preservatives, fragrances, etc.

### [1-3-1. Other base ingredients]

The composition for producing a capsule film may contain, together with the plant-derived protein, substrate components (other base ingredients) of typical capsule films. Other base ingredients can be animal-derived proteins such as gelatin, starch hydrolysates such as starch and dextrin, carrageenan, etc.

### [1-3-2. Solvents]

The composition for producing a capsule film may contain a solvent; and the solvent is preferably water but can contain an organic solvent such as ethanol, isopropyl alcohol, acetone, ethyl butyrate, etc. However, the content amount of the solvent (preferably, water) in the composition for producing a capsule film of the present invention relative to the total amount of the composition is preferably less than 20 mass%, more preferably less than 15 mass%, yet more preferably less than 10 mass%, and particularly preferably less than 5 mass%. To the composition for producing a capsule film, water does not have to be added except for the water that is blended in association with the blended material components, such as water contained in the raw materials of the plant-derived protein, etc.

By decreasing the content amount of water contained in the composition for producing a capsule film, a material having good formability for producing a capsule film is obtained. Furthermore, by decreasing the content amount of water, when a capsule film is produced from the material for producing a capsule film or when a capsule formulation constituted by a capsule film and a content is produced, it becomes possible to shorten or omit the drying step, which is a significant advantage.

Typically, when a composition containing a protein (for example, a composition for producing a capsule film containing as a main component gelatin which is an animal-based protein or a polysaccharide, or a composition containing a plant-derived protein) is subjected to pressurized heat treatment, a significant amount of water has been blended into the composition (for example, see Japanese Unexamined Patent Application Publication No. 2015-8642). In contrast, in the composition for producing a capsule film of the present invention, the content amount of water can be decreased.

### [1-3-3. Thickener]

The composition for producing a capsule film may contain a thickener; and examples of the thickener include gelatin, iota carrageenan, kappa carrageenan, lambda carrageenan, pullulan, agar, alginic acid, sodium alginate, alginic acid esters, gums, etc. The thickeners may be used alone or may be used in combination of two or more types.

### [1-3-4. pH adjuster]

The composition for producing a capsule film may contain a pH adjuster; and examples of the pH adjuster are, but are not particularly limited to, acids such as hydrochloric acid, sulfuric acid, nitric acid, etc.; bases such as sodium hydroxide, potassium hydroxide, etc.; and buffers such as phosphate buffers, tris buffers, etc. The pH adjusters can be used alone or can be used in combination of two or more types.

### [1-3-5. Metal sequestering agent]

Examples of the metal sequestering agent (also known as metal ion sequestering agent or chelating agent) are citric acid, tartaric acid, lactic acid, phosphoric acid, acetic acid, gluconic acid, ethylenediaminetetraacetic acid, metaphosphoric acid, salts thereof, glycine, etc. The metal sequestering agents can be used alone or can be used in combination of two or more types.

For other components also, substances that have been typically used as the capsule film components can be used.

### [1-4. Production of a composition for producing a capsule film]

The method for producing the composition for producing a capsule film is not particularly limited, and a known means can be used for production. For example, the production can involve mixing a plant-derived protein, a formability imparting component, and, if necessary, other components by using a mixer or the like. Mixing may be performed under heating or cooling if necessary.

### [2. Material for producing a capsule film]

A material for producing a capsule film according to one embodiment of the present invention can be obtained from a composition for producing a capsule film. More specifically, a material for producing a capsule film can be obtained by pressurizing and heating (performing pressurized heat treatment) a composition for producing a capsule film. By subjecting the composition for producing a capsule film to pressurized heat treatment, the plant-derived protein contained therein becomes indigestible, and a material having good formability for producing a capsule film can be produced.

Examples of the technique for pressurizing the composition for producing a capsule film are: A) a technique of kneading the composition for producing a capsule film; B) a technique of statically pressurizing the composition for producing a capsule film, etc.

Kneading as the technique of pressurizing means mixing the composition for producing a capsule film while applying pressure, and is different from simple mixing. The composition for producing a capsule film can be kneaded by using any desired kneader, and this kneader preferably has a kneading function and a heating function through screws, blades, rolls, etc. In other words, kneading of the composition for producing a capsule film can be melt-kneading under the heating condition. In addition, the composition for producing a capsule film is preferably kneaded by using an extruder preferably equipped with two screws (twin-screw-type extruder). A twin-screw-type extruder can continuously perform kneading and forming. The rotation rate of the screws when a twin-screw-type extruder is used to perform kneading is preferably 2 to 20 rpm and more preferably 4 to 16 rpm.

Static pressurization as the pressurizing technique involves pressurizing a statically placed assembly (bulk) of the composition for producing a capsule film. The composition for producing a capsule film can be statically pressurized by using, for example, a heat press machine. In this case, the press pressure is preferably 5 to 50 MPa and particularly preferably 10 to 30 MPa.

The lower limit of the temperature (heating temperature) in heating the composition for producing a capsule film is preferably 50°C, more preferably 70°C, and particularly preferably 115°C. Meanwhile, the upper limit of the temperature (heating temperature) in heating the composition for producing a capsule film is preferably 200°C, more preferably 190°C, and yet more preferably 180°C. By adjusting the heating temperature within such ranges, a material having good formability for producing a capsule film is obtained.

Furthermore, the temperature (heating temperature) in heating the composition for producing a capsule film can be set such that the plant-derived protein contained in the composition can become indigestible (denatured), and, for this, it is normally preferable to set the heating temperature to 120 to 160°C.

Note that when the composition for producing a capsule film is subjected to pressurized heat treatment with an extruder, the heating temperature means the temperature of the feeder (raw material injecting portion) or the barrel (a section where screws rotate inside to knead the raw material) of the extruder. Here, the extruder has a feeder (raw material injecting portion) and a barrel (a section where screws rotate inside to knead the raw material); and typically, the barrel is configured such that the heating temperature can be set for each of multiple regions divided along the direction from a part directly below the feeder to the extrusion exit. The heating temperature of the composition for producing a capsule film to be pressurized and heated by using an extruder means the heating temperature in at least one region of the aforementioned multiple regions, and it is not necessary that adjustment to this heating temperature be made in all regions.

The material for producing a capsule film is characterized by high formability; and, for example, the formability can be indicated by tensile strength. The material for producing a capsule film preferably has a tensile strength of 2.0 N/mm² or more and more preferably 3.0 N/mm² or more as measured by a testing method specified in JIS-K 7127; meanwhile, the tensile strength is preferably 10 N/mm² or less. Furthermore, in dynamic viscoelasticity measurement (measurement by a rotational rheometer), the storage modulus G' curve preferably has a rubbery plateau in the range of 70 to 170°C.

### [3. Capsule film and capsule formulation]

A capsule film according to one embodiment of the present invention can be a formed product of the material for producing a capsule film. In other words, as with the composition for producing a capsule film and the material for producing a capsule film, the capsule film contains the formability imparting component and the plant-derived protein, and, if necessary, can contain other components. Regarding the components and the content amounts thereof, the descriptions in "1. Composition for producing a capsule film" above apply equally. The plant-derived protein contained in the capsule film of the present invention is preferably a denatured product of a protein collected from a plant or a denatured product of a protein contained in a fermented product made from a plant as a raw material. The denatured product can be a heat denatured product, a pressure denatured product, or a heat-pressure denatured product.

A capsule film of the present invention can be a capsule film of a capsule formulation; and, in the capsule formulation, inside of the capsule film can be filled with (encapsulate) a content. The capsule formulation is preferably a soft capsule formulation, and can be filled with liquid-type or paste-type content.

The film thickness of the capsule film of the present invention is preferably 200 µm or more. This is to securely seal the content encapsulated in the capsule film. The upper limit of this film thickness is not particularly limited, but is usually several thousand µm or less. The film thickness of the capsule film can be measured with a digital microscope.

A capsule film of the present invention can be produced by forming a material for producing a capsule film. For example, the material for producing a capsule film of the present invention can be formed into a capsule film by injecting by an extruder the material for producing a capsule film into a desired capsule-shaped die, and then cooling the extrudate.

Alternatively, as another method for producing a capsule film, the material for producing a capsule film can be formed into a film shape, a tubular shape, a pellet shape, or the like while being extruded from the extruder; and then be formed into a capsule film by a known method. For example, when the material for producing a capsule film of the present invention is extrusion-formed into a film shape, the resulting product is subsequently formed into a capsule film by a punching method and then can be used to produce a capsule formulation. In addition, a capsule film can be produced by extrusion-forming the material for producing a capsule film of the present invention into a pellet shape and melting the pellet-shaped material for producing a capsule film; however, in such a case, the aforementioned [1-3. Other components] can be added to the melted product.

A capsule formulation (preferably a soft capsule formulation) including the capsule film of the present invention can be produced by a known method. In the capsule formulation, the content to be encapsulated in the capsule film is not particularly limited, and known active substances and additives can be selected. The shape of the capsule formulation is also not particularly limited, and can be any desired shape such as a spherical shape, an elliptic shape, etc.

The capsule formulation including the capsule film of the present invention contains a plant-derived protein in the capsule film, and thus various preferable physiological actions such as anti-inflammatory effects based on the plant-derived protein, etc., can be imparted to the capsule formulation.

In addition, the capsule formulation including the capsule film of the present invention can contain an indigestible plant-derived protein in the capsule film, and thus there is a possibility that the active substance contained in the content of the capsule formulation can be delivered to the large intestine and a large intestine-targeted DDS can be realized. In other words, there are cases in which the content of the capsule formulation preferably contain an active substance to be absorbed in the large intestine. Alternatively, since the capsule formulation including the capsule film of the present invention can contain an indigestible plant-derived protein in the capsule film, encapsulation of highly reactive content can be realized.

### [4. Method for reducing the digestibility of the protein]

As mentioned above, in one embodiment of the present invention, a plant-derived protein-containing composition for producing a capsule film is provided, and then by pressurizing and heat-treating the composition a material having indigestibility and good formability for producing a capsule film can be provided. Furthermore, the present invention also provides a method for reducing the digestibility of not only the plant-derived protein but also proteins in general (for example, animal-derived proteins etc.). The reduction in digestibility can be confirmed through a common artificial digestion assay. This assay can involve pressurizing and heat-treating a composition that contains a protein.

In a method for reducing digestibility of a protein, the composition containing the protein can contain the components similar to the formability imparting component in the composition for producing a capsule film, water, etc. The formability imparting component is preferably a polyol and more preferably glycerin. The ratio of the protein and the formability imparting component can also be set in the similar manner to the composition for producing a capsule film.

Pressurization and heat treatment of the composition containing a protein can be performed under the same conditions as the pressurization and heat treatment of the composition for producing a capsule film. In other words, a composition containing a protein is to be kneaded or treated by static pressurization; and a kneader such as a twin-screw-type extruder or the like, a heat press machine, etc., can be used.

A material obtained by pressurizing and heat-treating a composition containing a protein and a formability imparting component preferably has good formability as with the material for producing a capsule film described above; and can have, for example, a tensile strength similar to that of the material for producing a capsule film.

### EXAMPLES

The present invention will now be specifically described through examples. However, the present invention is not to be interpreted as being limited by the examples described below.

### [EXAMPLE 1]

### (Production of a composition for producing a capsule film and a material for producing a capsule film)

To 20 g of Wilpro N10 (produced by Wilmar International, protein content: 86.4 mass%, water content: 5.6 mass%) containing a soybean protein, 20 g of glycerin was added, and the resulting mixture was mixed to prepare a composition for producing a capsule film. Furthermore, the obtained composition for producing a capsule film was fed to a twin screw extruder, heated, and pressurized, and then the kneaded product was extruded from the barrel tip portion at a normal pressure to obtain a material for producing a capsule film. The barrel temperature was set to 90°C at the feeder portion (directly below the raw material injection portion), 130°C at the center portion, and 130°C at the tip portion. The screw rotation rate was set to 8 rpm. The obtained material for producing a capsule film exhibited a rubbery plateau in the range of 70 to 170°C, in which a dynamic viscoelasticity meter (Ares-G2 produced by Waters) was used to measure the temperature dependency of the storage modulus G' with conditions of a 25 mm parallel plate jig at an angular velocity of 6.28 rad/sec, a gap of 1 mm, and a heating rate of 0.1°C/sec.

### (Evaluation of formability)

The appearance properties of the extruded material for producing a capsule film was observed, and the formability was evaluated according to the following standard. The evaluation results are indicated in Table 1. Here, a material that takes a long continuous sheet shape can be continuously formed into capsule films and is evaluated as having good formability.
⊚: State in which the material takes a long and continuous sheet shape
∘: State in which the material takes a sheet shape but is short or thin, or takes a mass shape
Δ: Flake in shape
×: Powder in shape

### (Evaluation of digestibility)

Pancreatin (produced by Fujifilm Wako Pure Chemical Industries, Ltd.), a digestive enzyme, was dissolved in purified water having a pH adjusted to 9 by using 0.1 mol/L NaOH to prepare a testing liquid having a pancreatin concentration of 0.3 mass%. To 50 mL of the prepared testing liquid, 100 mg of the material for producing a capsule film was added, the resulting mixture was left to stand still at 45°C for 5 hours, and the residue was recovered by centrifugal separation and dried at 50°C for 16 hours. The dried residue was divided by the solid matter-based mass of the test specimen to determine as percentage. The evaluation results are indicated in Table 1. The larger the figure, the higher the indigestibility.

### [Examples 2 to 20], [Reference Example], [Comparative Examples 1 to 3]

Production of the material for producing a capsule film and evaluation therefor were carried out as in Example 1 except that the types and the added amounts of the components in Example 1 were changed as indicated in Tables 1 to 6. The evaluation results are indicated in Tables 1 to 6. Here, in Comparative Example 3, a simple mixture of Wilpro N10 and glycerin was evaluated for the digestibility.

The materials used in Examples 1 to 20, Reference Example, and Comparative Examples 1 to 3 are indicated below (see Tables 1 to 5).

### <Plant-derived protein>

· Wilpro N10: containing 86.4 mass% soybean protein, 5.6 mass% water, and 3.6 mass% hydrocarbons (produced by Wilmar)
· Wilpro P20: containing 86.9 mass% soybean protein, 5.7 mass% water, and 2.9 mass% hydrocarbons (produced by Wilmar)
· A-glu G: containing 74 mass% wheat protein (produced by GLICO NUTRITION CO., LTD.)

### <Animal-derived protein>

· Whey protein: DaiichiLacto EM-90 (produced by DAIICHI-KASEI CO., LTD.)

### <Formability imparting component>

· HPC: hydroxypropyl cellulose (CELNY SL produced by NIPPON SODA CO., LTD.)
· POEM J-2081V: stearic acid monoester of diglycerin (produced by RIKEN VITAMIN CO., LTD.)
· Betain: trimethylglycine

**[Table 1]**

| | | Examples | | | | |
|---|---|---|---|---|---|---|
| | | 1 | 2 | 3 | 4 | 5 |
| Formability imparting component | Glycerin | 50 | 50 | 50 | 50 | 50 |
| Plant-derived protein | Wilpro N10 | 50 | 50 | 50 | 50 | 50 |
| Formability imparting component plant-derived protein (mass ratio) | | 55:45 | 55:45 | 55:45 | 55:45 | 55:45 |
| Barrel temperature (°C) | Feeder portion | 90 | 40 | 80 | 105 | 105 |
| | Center portion | 130 | 70 | 110 | 150 | 170 |
| | Tip portion | 130 | 70 | 110 | 150 | 170 |
| Screw rotation rate (rpm) | | 8 | 8 | 8 | 8 | 8 |
| Evaluation | Formability | ⊚ | Δ | Δ | ○ | ○ |
| | Digestibility (%) | 64 | - | 48 | 68 | 6.9 |

**[Table 2]**

| | | | Examples | | | | |
|---|---|---|---|---|---|---|---|
| | | | 6 | 7 | 8 | 9 | 10 |
| Formability imparting component | Glycerin | Parts by mass | 45 | 45 | 45 | 45 | 45 |
| | Triacetin | | 10 | | | | |
| | Betaine | | | 10 | | | |
| | Propylene glycol | | | | 10 | | |
| | Trehalose | | | | | 10 | |
| | Maltitol | | | | | | 10 |
| Plant-derived protein | Wilpro N10 | | 45 | 45 | 45 | 45 | 45 |
| Formability imparting component plant-derived protein (mass ratio) | | | 59:41 | 59:41 | 59:41 | 59:41 | 59:41 |
| Barrel temperature (°C) | Feeder portion | | 90 | 90 | 90 | 90 | 90 |
| | Center portion | | 130 | 130 | 130 | 130 | 130 |
| | Tip portion | | 130 | 130 | 130 | 130 | 130 |
| Screw rotation rate (rpm) | | | 16 | 8 | 8 | 8 | 16 |
| Evaluation | Formability | | ⊚ | ⊚ | ⊚ | ○ | ○ |
| | Digestibility (%) | | - | - | - | - | - |

**[Table 3]**

| | | | Examples | | | | |
|---|---|---|---|---|---|---|---|
| | | | 11 | 12 | 13 | 14 | 15 |
| Formability imparting component | Glycerin | Parts by mass | 45 | 45 | 37.5 | 25 | 12.5 |
| | Xylitol | | 10 | | | | |
| | Erythritol | | | 10 | | | |
| Plant-derived protein | Wilpro N10 | | 45 | 45 | 50 | 50 | 50 |
| Water | | | | | 12.5 | 25 | 37.5 |
| Formability imparting component plant-derived protein (mass ratio) | | | 59:41 | 59:41 | 48:52 | 38:62 | 25:75 |
| Barrel temperature (°C) | Feeder portion | | 90 | 90 | 90 | 90 | 90 |
| | Center portion | | 130 | 130 | 130 | 130 | 130 |
| | Tip portion | | 130 | 130 | 130 | 130 | 130 |
| Screw rotation rate (rpm) | | | 8 | 4 | 8 | 8 | 8 |
| Evaluation | Formability | | ○ | ○ | ⊚ | ○ | ○ |
| | Digestibility (%) | | - | - | 53 | 57 | 70 |

**[Table 4]**

| Examples | | | Examples | | | | Reference Example |
|---|---|---|---|---|---|---|---|
| | | | 16 | 17 | 18 | 19 | |
| Formability imparting component | Glycerin | Parts by mass | 40 | 70 | 50 | 50 | 50 |
| Plant-derived protein | Wilpro N10 | | 60 | 30 | | | |
| | Wilpro P20 | | | | 50 | | |
| | A-glu G | | | | | 50 | |
| Animal-derived protein | Whey protein | | | | | | 50 |
| Formability imparting component plant-derived protein (mass ratio) | | | 45:55 | 73:27 | 54:46 | 57:43 | - |
| Barrel temperature (°C) | Feeder portion | | 90 | 90 | 90 | 90 | 90 |
| | Center portion | | 130 | 130 | 130 | 130 | 130 |
| | Tip portion | | 130 | 130 | 130 | 130 | 130 |
| Screw rotation rate (rpm) | | | 8 | 8 | 8 | 8 | 8 |
| Evaluation | Formability | | ⊚ | ⊚ | ⊚ | ⊚ | × |
| | Digestibility (%) | | - | - | 47 | 83 | 70 |

**[Table 5]**

| Example | | | 20 |
|---|---|---|---|
| Formability imparting component | Glycerin | Parts by mass | 49 |
| | HPC | | 20 |
| | POEM J-2081V | | 2 |
| Plant-derived protein | Wilpro N10 | | 29 |
| Formability imparting component plant-derived protein (mass ratio) | | | 74:26 |
| Barrel temperature (°C) | Feeder portion | | 100 |
| | Center portion | | 150 |
| | Tip portion | | 150 |
| Screw rotation rate (rpm) | | | 12 |
| Evaluation | Formability | | ⊚ |
| | Digestibility (%) | | - |

**[Table 6]**

| | | | Comparative Examples | | |
|---|---|---|---|---|---|
| | | | 1 | 2 | 3 |
| Formability imparting component | Glycerin | Parts by mass | 50 | | 50 |
| Plant-derived protein | Wilpro N10 | | 50 | 50 | 50 |
| Water | | | | 50 | |
| Formability imparting component plant-derived protein (mass ratio) | | | 55:45 | - | 55:45 |
| Barrel temperature (°C) | Feeder portion | | Room temperature | 90 | - |
| | Center portion | | | 130 | - |
| | Tip portion | | | 130 | - |
| Screw rotation rate (rpm) | | | 8 | 8 | - |
| Evaluation | Formability | | × | × | - |
| | Digestibility (%) | | - | 59 | 0.70 |

As indicated in Examples 1 to 5 (Table 1), when a composition containing, at a specified ratio, a plant-derived protein (soybean protein) and glycerin which was a formability imparting agent was subjected to pressurized heat treatment with a twin-screw-type extruder, the formability could be controlled by the heating temperature thereof; in particular, the formability was most excellent when the temperatures of the feeder portion, the center portion, and the tip portion of the extruder were respectively set to 90°C, 130°C, and 130°C. In addition, it is shown that the digestibility can also be controlled by the heating temperature. However, it was found that it was difficult to reduce the digestibility (make indigestible) when the temperatures of the feeder portion, the center portion, and the tip portion of the extruder were respectively set to 105°C, 170°C, and 170°C. As such, it can be understood that when the heating temperature in the pressurized heat treatment is excessively high, indigestibility is rarely achieved.

Meanwhile, as indicated in Comparative Example 1 (Table 6), when a composition containing, at a specified ratio, a plant-derived protein (soybean protein) and glycerin which was a formability imparting agent was treated with a twin-screw-type extruder without heating, a material with poor formability evaluation was obtained. Furthermore, as indicated in Comparative Example 3 (Table 6), a (untreated) composition containing, at a specified ratio, a plant-derived protein (soybean protein) and glycerin which was a formability imparting agent did not have reduced digestibility. Moreover, as indicated in Comparative Example 2 (Table 6), when a composition containing a plant-derived protein (soybean protein) and water was subjected to pressurized heat treatment with a twin-screw-type extruder, reduction in digestibility was observed, but a material with poor formability evaluation was obtained.

As indicated in Examples 6 to 12 (Tables 2 and 3), when a composition containing a plant-derived protein (soybean protein), glycerin as the main component of the formability imparting component, and various components (triacetin, betaine, propylene glycol, trehalose, maltitol, xylitol, and erythritol) was subjected to pressurized heat treatment with a twin-screw-type extruder, a material with sufficient formability was obtained in all examples.

As indicated in Examples 13 to 15 (Table 3), when a composition containing a plant-derived protein (soybean protein), glycerin which was a formability imparting component, and water was subjected to pressurized heat treatment with a twin-screw-type extruder, a material with good formability was obtained in all examples; however, the formability tended to decrease as the amount of water added increased. Meanwhile, it was found that the digestibility was likely to reduce (indigestibility was likely to improve) when the amount of water added increased.

As indicated in Examples 16 and 17 (Table 4), a material with good formability was still obtained when the content ratio of the plant-derived protein (soybean protein) and glycerin which was a formability imparting component was changed.

As indicated in Examples 18 and 19 and Reference Example (Table 4), when compositions containing a variety of proteins (soybean protein, wheat protein, and whey protein) and glycerin which was a formability imparting component were subjected to pressurized heat treatment with a twin-screw-type extruder, the digestibility was reduced in all examples; however, although a material with good formability was obtained in Examples 18 and 19, formability was poor in Reference Example. In Reference Example in which a whey protein (animal-based protein) was blended, it was considered that, compared to the plant-based protein, thermal aggregation barely occurred, thereby inhibiting improvement on the formability.

As indicated in Example 20 (Table 5), when a composition containing a plant-derived protein (soybean protein), and glycerin as the main component as well as HPC (hydroxypropyl cellulose) and POEM J-2081V (monoester of stearic acid and diglycerin), as the formability imparting component, was subjected to pressurized heat treatment with a twin-screw-type extruder, a material with good formability was obtained. This shows that a variety of formability imparting components can be used in combination.

### [EXAMPLE 21]

### (Production of a material for producing a capsule film by a heat press machine)

12 g of Wilpro N10 and 8 g of glycerin were mixed to prepare a composition for producing a capsule film. Furthermore, the obtained composition for producing a capsule film was fed to a heat press machine and was heated and pressurized under conditions of 15 MPa and 129°C for 120 seconds to produce a film having a thickness of about 0.8 mm.

### (Evaluation of digestibility and tensile strength)

The obtained film was evaluated for digestibility as in Example 1, and the result was 70%. Furthermore, the tensile strength of the obtained film was measured by referring to Plastics - Determination of tensile properties (JIS K7127), and the result was 3.28 ± 0.34 N/mm². The tensile strength of a conventional capsule film made from carrageenan as the raw material as measured by the same method was 1.61 ± 0.09 N/mm².

### (Production of a capsule film and a capsule formulation)

The film obtained as described above was formed into a pocket shape using a die to obtain two capsule films. One of the obtained capsule films was filled with a content (medium-chain fatty acid triglyceride) by hand, and a different die was used to join the two upper and lower capsule films. When joining, heating was carried out at 140°C for 10 seconds. No leakage of the content was observed from the obtained capsule formulation.

### Industrial Applicability

A capsule formulation having a novel function can be provided by the composition for producing a capsule film of the present invention, and the use applications of the capsule formulation can be widened. Specifically, for example, biological effects can be imparted to the capsule film itself; the large intestine drug delivery function (DDS function) can be imparted; and the content of the capsule can be a highly active drug.

## Claims

1. A composition for producing a capsule film, the composition comprising at least one type selected from the group consisting of polyols, triesters, and trialkylamino acids, and a plant-derived protein.

2. The composition according to Claim 1, wherein a content mass ratio of the at least one type selected from the group consisting of polyols, triesters, and trialkylamino acids to the plant-derived protein is 25:75 to 80:20.

3. The composition according to Claim 1 or 2, wherein a content ratio of water is less than 20 mass%.

4. The composition according to Claim 1 or 2, wherein the plant-derived protein includes at least one type selected from the group consisting of soybean-derived proteins, adzuki bean-derived proteins, kidney bean-derived proteins, cacao bean-derived proteins, wheat-derived proteins, buckwheat-derived proteins, and rice-derived proteins.

5. The composition according to Claim 1 or 2, wherein the at least one type selected from the group consisting of polyols, triesters, and trialkylamino acids includes glycerin.

6. A method for producing a material for producing a capsule film, the method comprising a step of pressurizing and heating a composition containing at least one type selected from the group consisting of polyols, triesters, and trialkylamino acids, and a plant-derived protein.

7. The method for producing according to Claim 6, wherein a content mass ratio of the at least one type selected from the group consisting of polyols, triesters, and trialkylamino acids to the plant-derived protein is 25:75 to 80:20.

8. The method for producing according to Claim 6 or 7, wherein a content ratio of water in the composition is less than 20 mass%.

9. The method for producing according to Claim 6 or 7, wherein the plant-derived protein includes at least one type selected from the group consisting of soybean-derived proteins, adzuki bean-derived proteins, kidney bean-derived proteins, cacao bean-derived proteins, wheat-derived proteins, buckwheat-derived proteins, and rice-derived proteins.

10. The method for producing according to Claim 6 or 7, wherein the at least one type selected from the group consisting of polyols, triesters, and trialkylamino acids includes glycerin.

11. The method for producing according to Claim 6 or 7, wherein the heating temperature is 50 to 200°C.

12. A capsule film comprising at least one type selected from the group consisting of polyols, triesters, and trialkylamino acids, and a plant-derived protein.

13. The capsule film according to Claim 12, wherein a content mass ratio of the at least one type selected from the group consisting of polyols, triesters, and trialkylamino acids to the plant-derived protein is 25:75 to 80:20.

14. The capsule film according to Claim 12 or 13, wherein the plant-derived protein includes at least one type selected from the group consisting of soybean-derived proteins, adzuki bean-derived proteins, kidney bean-derived proteins, cacao bean-derived proteins, wheat-derived proteins, buckwheat-derived proteins, and rice-derived proteins.

15. The capsule film according to Claim 12 or 13, wherein the at least one type selected from the group consisting of polyols, triesters, and trialkylamino acids includes glycerin.

16. The capsule film according to Claim 12 or 13, wherein the plant-derived protein has been subjected to pressurized heat treatment.

17. A capsule formulation comprising the capsule film according to Claim 12 or 13, and a content encapsulated in the capsule film.

18. A method for reducing digestibility of a protein, the method comprising a step of pressurizing and heating a composition containing the protein.

19. The method according to Claim 18, wherein the composition further contains a polyol.
